# EUROPEAN PATENT APPLICATION

(11) **EP 4 613 255 A1**
(43) Date of publication of application: **10.09.2025**
(21) Application number: 23911412.7
(22) Date of filing: 13.11.2023
(51) Int. Cl.: A61J 1/20, A61M 5/142, A61M 5/32

(54) **VIAL ADAPTER AND CARTRIDGE UNIT INCLUDING SAME**

(30) Priority: 26.12.2022 JP 2022207803
(71) Applicant: Terumo Kabushiki Kaisha, Tokyo 151-0072 (JP)
(72) Inventor: NAKAI, Masahiro, Nakakoma-gun, Yamanashi 409-3853 (JP); UCHIYAMA, Joji, Ashigarakami-gun, Kanagawa 259-0151 (JP); SUGIYAMA, Takayuki, Nakakoma-gun, Yamanashi 409-3853 (JP); HEIKKINEN, Tero, 90230 Oulu (FI); PAETSI, Matti, 90230 Oulu (FI); HEIKKILAE, Mikael, 90230 Oulu (FI); KALLUNKI, Markku, 90230 Oulu (FI)
(74) Representative: TBK
(86) International application number: PCT/JP2023/040757
(87) International publication number: WO 2024/142629

(57) **Abstract**

A vial adapter (10) includes a vial attachment portion (12) to which a vial (150) is attached, and a device attachment portion (14) to which a liquid medication administration device (100) is attached. Liquid medication (L) in the vial is delivered from the device attachment portion to a reservoir (102) of the liquid medication administration device via a liquid delivery tube (40). The liquid delivery tube has a protrusion portion (40a) protruding from the device attachment portion. On the other hand, the device attachment portion includes a groove (46). The device attachment portion is able to be folded with the groove as a folding point. As the device attachment portion is folded, the protrusion portion of the liquid delivery tube is shielded by the device attachment portion.

## Description

### Technical Field

The present invention relates to a vial adapter for transferring liquid medication from a vial to a reservoir. In addition, the present invention also relates to a cartridge unit including a vial adapter and a cartridge constituting a liquid medication administration device.

### Background Art

A portable liquid medication administration device includes a housing and a reservoir provided in the housing. Before use of the liquid medication administration device, the reservoir is not filled with the liquid medication. Therefore, a user needs to transfer an appropriate amount of liquid medication from the vial to the reservoir prior to use of the liquid medication administration device.

As disclosed in WO 2016/132936, the vial and the liquid medication administration device are connected to each other via the vial adapter. The liquid medication in the vial is moved to the reservoir via the vial adapter. Specifically, the vial adapter has a liquid delivery tube. The liquid medication in the vial flows through the liquid delivery tube and flows into the reservoir. The liquid medication filled in the reservoir is administered to a patient.

### Summary of Invention

After removing the vial adapter from the liquid medication administration device, the patient uses the liquid medication administration device by, for example, attaching the liquid medication administration device to the skin. When the vial adapter is removed from the liquid medication administration device, the distal end of the liquid delivery tube is exposed. Therefore, there is a concern that the distal end of the liquid delivery tube comes into contact with the user or the like.

In addition, the vial adapter from which the liquid medication administration device is removed is disposed of as medical waste. That is, the vial adapter is discarded each time the patient replaces the liquid medication administration device. Therefore, in a case where a plurality of vial adapters is discarded at the same time, the accumulation of the plurality of vial adapters results in the formation of bulky medical waste.

An object of the present invention is to solve the above problems.

(1) An aspect of the present invention is a vial adapter for transferring liquid medication in a vial to a reservoir of a liquid medication administration device, the vial adapter including a vial attachment portion to which a vial is attached; and a device attachment portion that extends from the vial attachment portion and to which the liquid medication administration device is attached; a liquid delivery tube that has a protrusion portion protruding from the device attachment portion and delivers the liquid medication from the vial to the reservoir; and a groove that is formed in the device attachment portion and extends in a direction orthogonal to an attachment direction of the vial to the vial attachment portion, in which the device attachment portion is able to be folded with the groove as a folding point, and when the device attachment portion is folded, the protrusion portion of the liquid delivery tube is shielded by the device attachment portion.
   In this configuration, the device attachment portion can be folded as described above. At the time of this folding, the liquid delivery tube is shielded. Therefore, a distal end of the liquid delivery tube is prevented from puncturing a user. Therefore, the user can easily handle the vial adapter. In addition, since the vial adapter can be folded, the vial adapter after use can be discarded as compact waste (medical waste).
(2) In the above item (1), when the vial adapter is divided, with the groove as a boundary, into a first segment including the vial attachment portion and a second segment not including the vial attachment portion, the first segment includes a first stopper portion and the second segment includes a second stopper portion. In this configuration, when the device attachment portion is folded such that the first segment approaches the second segment, the first stopper portion and the second stopper portion are engaged with each other.
   By this engagement, the folded device attachment portion is prevented from returning to an original shape before being folded. Therefore, a state in which the liquid delivery tube is shielded is maintained. This makes it easier for the user to handle the vial adapter. In addition, for the vial adapter after use, a compact medical waste state can be maintained.
(3) In the above item (1) or (2), the vial adapter may include an air supply needle for supplying air to the vial, the device attachment portion may include a peripheral wall portion covering the air supply needle, and a slit continuous with the groove may be formed in the peripheral wall portion.
   In this case, the slit is formed in the peripheral wall portion which is a part of the device attachment portion. When the device attachment portion is folded with the groove as the folding point, the slit expands. Therefore, the folding of the device attachment portion is not hindered.
(4) In any one of the above items (1) to (3), the vial adapter may include a recess portion that accommodates the protrusion portion of the liquid delivery tube when the device attachment portion is folded.
   Since the protrusion portion of the liquid delivery tube enters the recess portion, the protrusion of the protrusion portion outward from the device attachment portion is avoided.
(5) In any one of the above items (1) to (4), the device attachment portion may include an abutting portion that abuts on the liquid medication administration device. In this case, the groove may be formed in the abutting portion.
   Since the length of the groove is increased, it is easier to fold the device attachment portion.
(6) In the above item (5), the abutting portion includes a flat portion and a curved portion. In this case, when the device attachment portion is folded, the flat portion and the curved portion face each other, and the curved portion covers the protrusion portion of the liquid delivery tube.
   Since the protrusion portion is covered with the curved portion, the liquid delivery tube is further prevented from puncturing the user.
(7) In any one of the above items (1) to (6), the device attachment portion may include a protrusion for engagement.
   In this case, the liquid medication administration device is provided with a hole with which the protrusion is engaged. By the engagement via the protrusion and the hole, the liquid medication administration device can be easily attached to the device attachment portion. Moreover, the attached state is maintained. In other words, the liquid medication administration device is held by the device attachment portion.
(8) In any one of the above items (1) to (7), the device attachment portion may include a seal portion formed to cover an opening that is formed in the liquid medication administration device and is for projecting a cannula to puncture a patient.
   Even in a case where the liquid medication leaks from the reservoir and flows into the cannula, the seal portion closes the opening. Therefore, leakage of the liquid medication to the outside of the liquid medication administration device is avoided.
(9) Another aspect of the present invention is a cartridge unit including a vial adapter; and a cartridge constituting a liquid medication administration device in which a reservoir is accommodated, in which the vial adapter includes a vial attachment portion to which a vial containing liquid medication is attached, a device attachment portion that extends from the vial attachment portion and to which the cartridge is attached, a liquid delivery tube that has a protrusion portion protruding from the device attachment portion and delivers the liquid medication from the vial to the reservoir, a groove that is formed in the device attachment portion and extends in a direction orthogonal to an attachment direction of the vial to the vial attachment portion, and a first engagement portion provided in the device attachment portion, the cartridge includes a second engagement portion, one of the first engagement portion and the second engagement portion is an engagement protrusion, the other of the first engagement portion and the second engagement portion is an engagement hole, and the engagement protrusion is inserted into the engagement hole.
   By the engagement, the vial adapter is prevented from falling out of the reservoir. Therefore, it is easy to transfer the liquid medication in the vial to the reservoir.
(10) In the above item (9), the first engagement portion and the second engagement portion may be bonded to each other.
   By the bonding, the vial adapter is further prevented from falling out of the reservoir.
(11) In the above item (10), the first engagement portion and the second engagement portion may be welded to each other.
   The bonding strength by the welding is not so large. Therefore, after the transfer of the liquid medication from the vial to the reservoir is ended, the vial adapter can be easily removed from the cartridge. Accordingly, each of the vial adapter and the reservoir can be discarded individually. Note that, as a preferable specific example of the welding, ultrasonic bonding is exemplified.
(12) In any one of the above items (9) to (11), the cartridge may include a cannula that punctures a patient to deliver the liquid medication from the reservoir to the patient, and an opening for projecting the cannula. The device attachment portion may include a seal portion that covers the opening when the engagement protrusion is inserted into the engagement hole.

Since the seal portion closes the opening, as in the above item (8), even in a case where the liquid medication leaks from the reservoir and flows into the cannula, leakage of the liquid medication to the outside of the liquid medication administration device is avoided.

According to the present invention, since it is possible to shield the liquid delivery tube by folding the vial adapter, the distal end of the liquid delivery tube is prevented from puncturing the user. Therefore, the user can easily handle the vial adapter. In addition, by folding the vial adapter after use, the vial adapter can be discarded as compact medical waste.

### Brief Description of Drawings

Fig. 1 is a schematic perspective view of a filling device for transferring liquid medication in a vial to a liquid medication administration device.
Fig. 2 is an overall schematic perspective view of a vial adapter according to an embodiment of the present invention.
Fig. 3 is an exploded perspective view illustrating a state before a vial adapter is attached to a liquid medication administration device.
Fig. 4 is a perspective view illustrating a state after a vial adapter is attached to a liquid medication administration device.
Fig. 5 is an exploded perspective view illustrating a state in which a vial adapter is being removed from a liquid medication administration device.
Fig. 6 is a schematic perspective view illustrating a state where a vial adapter is being folded.
Fig. 7 is an overall schematic perspective view of a folded vial adapter.
Fig. 8 is an enlarged cross-sectional view of a main part illustrating a state where a second stopper portion is bent by a first stopper portion pushing the second stopper portion.

### Description of Embodiments

Hereinafter, a "user" refers to a person who fills a reservoir 102 of a liquid medication administration device 100 with liquid medication L. A typical example of the user is a patient, but the user is not limited to the patient.

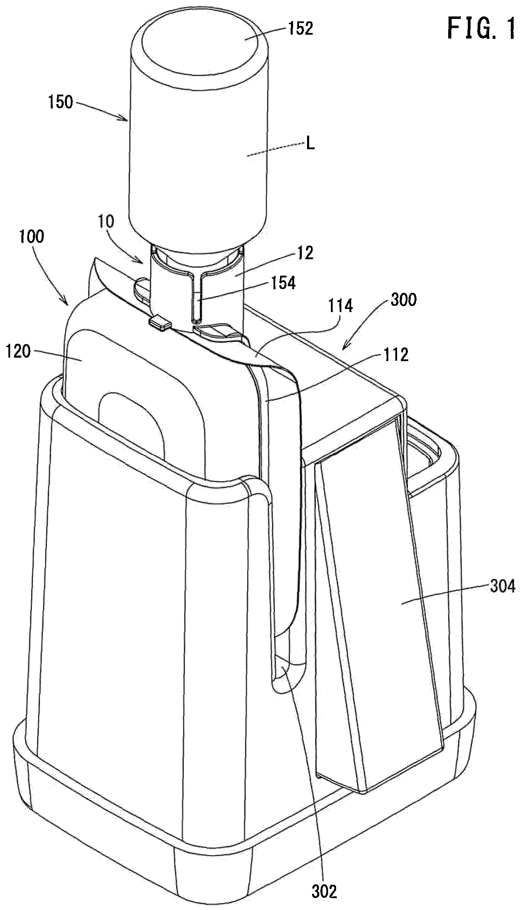

Fig. 1 is a schematic perspective view of a filling device 300. The filling device 300 is formed with a loading slot 302 extending along a vertical direction. The liquid medication administration device 100 is inserted into the loading slot 302. With this insertion, the liquid medication administration device 100 is loaded into the filling device 300. Note that a vial 150 containing the liquid medication is connected in advance to the liquid medication administration device 100 via a vial adapter 10. The inlet/outlet of the vial 150 faces downward and a bottom portion 152 of the vial 150 faces upward. That is, the vial 150 is in an inverted position.

The inlet/outlet of the vial 150 is sealed with a plug member 154. The plug member 154 may have a self-sealing ability. The plug member 154 is, for example, a septum such as a rubber plug. Note that in this aspect, the vial 150 having a so-called bottle shape is exemplified, but the vial 150 is not limited thereto. For example, the vial 150 may be a cartridge for an insulin pen.

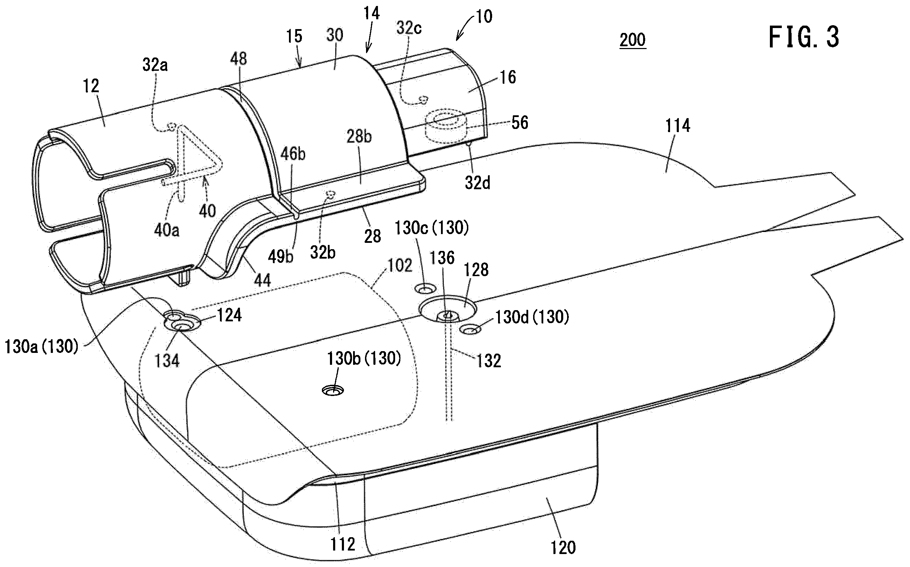

The filling device 300 includes an air supply port (not illustrated) and a lever 304. As the user pushes the lever 304, a predetermined amount of air is compressed and discharged from the air supply port. The compressed air is supplied to the vial 150 via the vial adapter 10. The liquid medication L in the vial 150 is pushed out of the vial 150 by the compressed air. The liquid medication L pushed out of the vial 150 is moved to the reservoir 102 (refer to Fig. 3) of the liquid medication administration device 100 via the vial adapter 10.

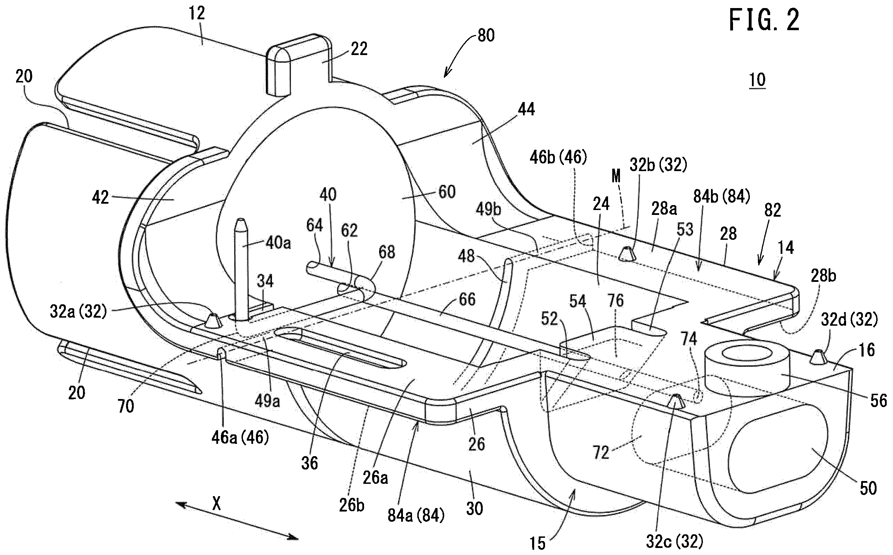

A specific configuration of the vial adapter 10 will be described. Fig. 2 is an overall schematic perspective view of the vial adapter 10. The X direction illustrated in Fig. 2 is an axial direction of the vial adapter 10, and is an attachment direction and a detachment direction of the vial 150 to and from a vial attachment portion 12. The vial adapter 10 has the vial attachment portion 12 and a device attachment portion 14. The vial attachment portion 12 and the device attachment portion 14 constitute an adapter main body. The vial adapter 10 further includes a liquid delivery tube 40 and an air supply needle 66.

A plurality of large notches 20 extending along the X direction is formed in the vial attachment portion 12. The plurality of large notches 20 relatively reduces rigidity of the vial attachment portion 12. Therefore, in the vial 150, it is easy to insert one end portion provided with the inlet/outlet into the vial attachment portion 12.

In the vial attachment portion 12, a first stopper portion 22 is provided at an end portion facing the device attachment portion 14. The first stopper portion 22 protrudes from the outer peripheral wall of the vial attachment portion 12 along a radial direction of the vial attachment portion 12.

The device attachment portion 14 includes a semi-cylindrical portion 15 and a socket portion 16. The semi-cylindrical portion 15 is a portion positioned between the vial attachment portion 12 and the socket portion 16. That is, the vial attachment portion 12, the semi-cylindrical portion 15, and the socket portion 16 are arranged along the X direction (the axial direction of the vial adapter 10).

In the device attachment portion 14, the semi-cylindrical portion 15 has a shape in which a cylinder is cut in substantially half along the X direction. That is, in this aspect, the semi-cylindrical portion 15 has a semi-cylindrical shape having an open space 24. Note that the semi-cylindrical portion 15 is not particularly limited to a semi-cylindrical shape. The semi-cylindrical portion 15 may have a shape in which a polygonal cylinder (for example, a quadrangular cylinder) is cut in substantially half along the X direction.

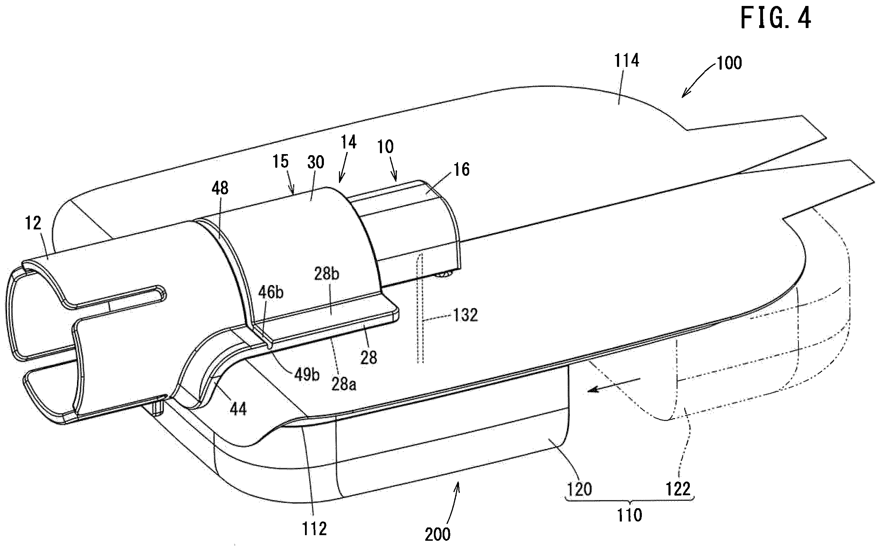

As understood from Figs. 3 and 4, when the vial adapter 10 is attached to the liquid medication administration device 100, the liquid medication administration device 100 covers the open space 24. Hereinafter, when the vial adapter 10 is attached to the liquid medication administration device 100, in the vial adapter 10, a surface facing the liquid medication administration device 100 is referred to as an inner surface, and a side opposite to the inner surface is referred to as an outer surface.

The semi-cylindrical portion 15 has a peripheral wall portion 30 that covers and protects the outer surface side of the air supply needle 66. The semi-cylindrical portion 15 further includes a first flat portion 26 and a second flat portion 28. The first flat portion 26 and the second flat portion 28 are wide portions protruding outward in the radial direction of the peripheral wall portion 30 from the end portions of the peripheral wall portion 30 in a circumferential direction. When the semi-cylindrical portion 15 is attached to the liquid medication administration device 100, the first flat portion 26 and the second flat portion 28 abut on a flat end surface of the liquid medication administration device 100.

An outer surface 26b of the first flat portion 26 is provided with a first groove 46a. The first groove 46a is recessed toward an inner surface 26a of the first flat portion 26. Similarly, an outer surface 28b of the second flat portion 28 is provided with a second groove 46b recessed toward an inner surface 28a of the second flat portion 28. The first groove 46a and the second groove 46b constitute a groove 46. The first groove 46a and the second groove 46b are positioned on an imaginary straight line M extending in a direction orthogonal to the X direction. Therefore, the first groove 46a and the second groove 46b are arranged on a straight line. The first groove 46a and the second groove 46b extend linearly along the imaginary straight line M.

In addition, the peripheral wall portion 30 is formed with a slit 48 penetrating from the inner surface to the outer surface of the peripheral wall portion 30. On the outer surface of the device attachment portion 14, one end of the slit 48 is continuous with the first groove 46a, and the other end of the slit 48 is continuous with the second groove 46b. The slit 48 extends in the circumferential direction of the peripheral wall portion 30. Therefore, when the inner surface side of the vial adapter 10 is viewed in plan view, the first groove 46a, the slit 48, and the second groove 46b are positioned on the imaginary straight line M. That is, the first groove 46a, the slit 48, and the second groove 46b extend in a direction orthogonal to the X direction. As described above, the X direction is the attachment direction and the detachment direction of the vial 150 with respect to the vial attachment portion 12.

In the first flat portion 26, a portion where the first groove 46a is formed is thinner than other portions. In the second flat portion 28, a portion where the second groove 46b is formed is thinner than other portions. In other words, the first flat portion 26 has a first thin portion 49a, and the second flat portion 28 has a second thin portion 49b. Therefore, the first thin portion 49a and the second thin portion 49b can be easily folded using the first groove 46a and the second groove 46b as folding points. Furthermore, in the peripheral wall portion 30 of the semi-cylindrical portion 15, the slit 48 is positioned between the first groove 46a and the second groove 46b. Therefore, it is also easy to fold the peripheral wall portion 30 of the semi-cylindrical portion 15.

The vial adapter 10 has a first portion and a second portion. The first portion and the second portion are divided with the first groove 46a, the slit 48, and the second groove 46b (imaginary straight line M) as boundaries. The first portion is a first segment 80 that includes the vial attachment portion 12 and a part of the device attachment portion 14 (a part of the semi-cylindrical portion 15, a first curved portion 42, and a second curved portion 44). The second portion is a second segment 82 including the majority of the semi-cylindrical portion 15 and the socket portion 16.

A first engagement protrusion 32a is provided on the inner surface of the first flat portion 26, and a second engagement protrusion 32b is provided on the inner surface of the second flat portion 28. The first engagement protrusion 32a and the second engagement protrusion 32b constitute a first engagement portion 32. The first engagement protrusion 32a and the second engagement protrusion 32b protrude toward the liquid medication administration device 100. The first engagement protrusion 32a is positioned in the first segment 80, and the second engagement protrusion 32b is positioned in the second segment 82.

A needle groove 34 is formed on the inner surface of the first segment 80 in the first flat portion 26. A recess portion 36 is formed on the inner surface of the second segment 82 in the first flat portion 26. The recess portion 36 is recessed from the inner surface toward the outer surface of the first flat portion 26. The liquid delivery tube 40 to be described later is inserted into the needle groove 34. Note that the first engagement protrusion 32a is provided in the vicinity of the needle groove 34 to be outward of the needle groove 34 in the radial direction.

The semi-cylindrical portion 15 further includes the first curved portion 42 and the second curved portion 44. The first curved portion 42 and the second curved portion 44 are positioned in the first segment 80. However, when the liquid medication administration device 100 is attached to the device attachment portion 14, the first curved portion 42 and the second curved portion 44 abut on the curved portion and the side surface of the liquid medication administration device 100 (refer to Figs. 3 and 4).

The first flat portion 26 and the first curved portion 42 constitute a first abutting portion 84a. The second flat portion 28 and the second curved portion 44 constitute a second abutting portion 84b. The inner surface of each of the first abutting portion 84a and the second abutting portion 84b abuts on the liquid medication administration device 100. That is, each of the first abutting portion 84a and the second abutting portion 84b is a part of an abutting portion 84. The first curved portion 42 and the second curved portion 44 are connected to the side surface of the end portion of the vial attachment portion 12, which faces the device attachment portion 14.

As illustrated in Fig. 2, the socket portion 16 extends from the end portion of the semi-cylindrical portion 15 along the X direction. A seal portion 56 is provided at an end portion of the socket portion 16 on a side away from the semi-cylindrical portion 15. A third engagement protrusion 32c and a fourth engagement protrusion 32d are further provided at the end portion. The seal portion 56 is positioned between the third engagement protrusion 32c and the fourth engagement protrusion 32d. The seal portion 56, the third engagement protrusion 32c, and the fourth engagement protrusion 32d protrude toward the liquid medication administration device 100. The third engagement protrusion 32c and the fourth engagement protrusion 32d constitute the first engagement portion 32.

A tube insertion hole 50 is formed at the end portion of the socket portion 16 on a side away from the semi-cylindrical portion 15. A supply tube (not illustrated) is inserted into the tube insertion hole 50. The supply tube is provided in the filling device 300. The air compressed by the user pushing the lever 304 flows into the tube insertion hole 50 via the supply tube. A bottom portion of the tube insertion hole 50 is a closure wall 72. A needle engagement hole 74 is formed in the closure wall 72. The needle engagement hole 74 penetrates the closure wall 72.

A recessed space 76 and two small notches 52 and 53 are formed at the end portion of the socket portion 16, which faces the semi-cylindrical portion 15. The two small notches 52 and 53 are continuous with the recessed space 76. As a result, a second stopper portion 54 exhibiting flexibility is provided between the two small notches 52 and 53.

As illustrated in Fig. 2, a partition wall 60 is provided between the vial attachment portion 12 and the semi-cylindrical portion 15. An upper surface of the partition wall 60 receives the plug member 154.

A first insertion hole 62 and a second insertion hole 64 are formed in the partition wall 60. One end portion of the air supply needle 66 extending along the X direction passes through the first insertion hole 62. When the vial 150 is attached to the vial attachment portion 12, one end portion of the air supply needle 66 includes a blade edge, and penetrates the plug member 154 to be positioned in the vial 150. The other end portion of the air supply needle 66 is inserted into the needle engagement hole 74 formed in the closure wall 72 of the socket portion 16. The other end portion of the air supply needle 66 may protrude into the tube insertion hole 50. Here, the supply tube is inserted into the tube insertion hole 50 of the socket portion 16. Therefore, the air supplied from the supply tube flows in from the other end portion of the air supply needle 66. The air supply needle 66 is a needle for supplying compressed air to the vial 150.

One end portion of the liquid delivery tube 40 is inserted into the second insertion hole 64. When the vial 150 is attached to the vial attachment portion 12, one end portion of the liquid delivery tube 40 penetrates the plug member 154 to be positioned in the vial 150. The liquid delivery tube 40 is a needle for transferring the liquid medication L from the vial 150 to the reservoir 102. The liquid delivery tube 40 includes a first crank 68 and a second crank 70. The second crank 70 is positioned in the needle groove 34. The liquid delivery tube 40 comes out of the partition wall 60 to extend substantially parallel to the air supply needle 66, is folded by the first crank 68, and extends toward the first flat portion 26 of the semi-cylindrical portion 15. Furthermore, the liquid delivery tube 40 is folded by the second crank 70 in the needle groove 34. The liquid delivery tube 40 has a needle distal end 40a protruding from the device attachment portion 14 (specifically, the first flat portion 26) at the distal end. The needle distal end 40a is a protrusion portion protruding from the device attachment portion 14. The needle distal end 40a extends toward the liquid medication administration device 100. The needle distal end 40a is positioned in the first segment 80 and faces the inner surface of the first curved portion 42.

As illustrated in Figs. 3 and 4, the vial adapter 10 is attached to the liquid medication administration device 100. The liquid medication administration device 100 will be described.

The liquid medication administration device 100 includes a device main body 110 (refer to Fig. 4) and an adhesive sheet 112 covering the device main body 110. In the liquid medication administration device 100 before use, the adhesive sheet 112 is covered with a release liner 114.

The device main body 110 includes a cartridge 120 and a pump unit 122 illustrated in Fig. 4. The pump unit 122 is attachable to and detachable from the cartridge 120 to which the adhesive sheet 112 is affixed. That is, the adhesive sheet 112 is not affixed to the pump unit 122.

A first through-hole 124 and a second through-hole 128 are formed in the cartridge 120, the adhesive sheet 112, and the release liner 114. In addition, in the cartridge 120, the adhesive sheet 112, and the release liner 114, first engagement hole 130a to fourth engagement hole 130d are respectively formed at positions corresponding to the first engagement protrusion 32a to the fourth engagement protrusion 32d. The first engagement hole 130a to the fourth engagement hole 130d constitute a second engagement portion 130.

The reservoir 102 and a cannula 132 are accommodated in the cartridge 120. The cartridge 120 and the reservoir 102 are transparent or translucent. Therefore, the user can visually check the amount of the liquid medication L stored in the reservoir 102. The reservoir 102 has a liquid medication inlet 134 and a liquid medication outlet (not illustrated). The liquid medication inlet 134 overlaps the first through-hole 124. One end portion of a tube (not illustrated) is connected to the liquid medication outlet. The other end portion of the tube is connected to the cannula 132.

The cartridge 120 has an opening 136. The cannula 132 protrudes from the opening 136. The opening 136 overlaps the second through-hole 128.

Before use of the liquid medication administration device 100, the entire cannula 132 is accommodated inside the device main body 110. When the liquid medication administration device 100 is used, the user performs a predetermined operation using a puncture device (not illustrated), whereby the cannula 132 advances from the opening 136 and the second through-hole 128 to puncture the patient's body.

Hereinafter, an assembly of the vial adapter 10 and the cartridge 120 provided with the adhesive sheet 112 and the release liner 114 is referred to as a cartridge unit 200. The assembling operation of the cartridge unit 200 is performed as follows.

As illustrated in Figs. 3 and 4, the cartridge 120 to which the adhesive sheet 112 is affixed is attached to the vial adapter 10. At the time of this attachment, the open space 24 in the semi-cylindrical portion 15 of the vial adapter 10 is covered with the release liner 114, and the first flat portion 26, the second flat portion 28, the first curved portion 42, and the second curved portion 44 abut on the release liner 114. In other words, the open space 24 of the semi-cylindrical portion 15 is closed by the liquid medication administration device 100, and the first abutting portion 84a and the second abutting portion 84b abut on the liquid medication administration device 100.

In addition, the needle distal end 40a of the liquid delivery tube 40 is inserted into the liquid medication inlet 134 via the first through-hole 124. The seal portion 56 provided in the socket portion 16 closes (seals) the opening 136 and the second through-hole 128. Furthermore, the first engagement protrusion 32a to the fourth engagement protrusion 32d are inserted into the first engagement hole 130a to the fourth engagement hole 130d, respectively.

Next, the first engagement protrusion 32a to the fourth engagement protrusion 32d are bonded to the inner walls of the first engagement hole 130a to the fourth engagement hole 130d, respectively. As described above, the vial adapter 10 is attached to the cartridge 120 in a separable manner, and is assembled as the cartridge unit 200. In the cartridge unit 200, the adhesive sheet 112 and the release liner 114 are interposed between the cartridge 120 and the device attachment portion 14. The cartridge unit 200 in this state can be packed in a packaging bag.

As described later, the user needs to remove the vial adapter 10 from the liquid medication administration device 100 before using the liquid medication administration device 100. Therefore, the bonding strength between the first engagement portion 32 and the second engagement portion 130 is preferably set so that the vial adapter 10 can be removed from the liquid medication administration device 100 by the manual operation of the user. In this case, the bonding between the first engagement portion 32 (the first engagement protrusion 32a to the fourth engagement protrusion 32d) and the second engagement portion 130 (the first engagement hole 130a to the fourth engagement hole 130d) can be released by the manual operation of the user.

As a preferable bonding method of the first engagement portion 32 and the second engagement portion 130, welding by ultrasonic bonding can be exemplified. In a case where no external force is applied to the vial adapter 10, the first engagement protrusion 32a to the fourth engagement protrusion 32d are prevented from being detached from the first engagement hole 130a to the fourth engagement hole 130d, respectively. Since the first engagement portion 32 and the second engagement portion 130 are arranged in a dispersed manner at a plurality of points, the bonding strength obtained by ultrasonic bonding is not so large. Therefore, in a case where a predetermined external force or more is applied to the vial adapter 10, the first engagement protrusion 32a to the fourth engagement protrusion 32d are easily detached from the first engagement hole 130a to the fourth engagement hole 130d, respectively.

Instead of ultrasonic bonding, the first engagement portion 32 and the second engagement portion 130 may be bonded with an adhesive having low adhesive strength. Alternatively, the first engagement protrusion 32a to the fourth engagement protrusion 32d may be fitted to the first engagement hole 130a to the fourth engagement hole 130d, respectively.

By applying these methods to the bonding or adhesion of the first engagement portion 32 and the second engagement portion 130, it becomes unnecessary to separately provide a fitting portion, an engagement section, or the like between the vial adapter 10 and the cartridge 120. Therefore, it is possible to further reduce the size and weight of the cartridge unit 200.

The liquid medication administration device 100 is used by the user as follows.

In order to make the liquid medication administration device 100 usable, the reservoir 102 in the cartridge 120 is filled with the liquid medication L. Therefore, the user attaches the pump unit 122 illustrated in Fig. 4 to the cartridge 120. Thus, the device main body 110 is configured.

Next, the user inserts the liquid medication administration device 100 to which the vial adapter 10 is attached, into the loading slot 302 of the filling device 300 (refer to Fig. 1). With this insertion, the supply tube provided in the filling device 300 is inserted into the tube insertion hole 50 of the socket portion 16. Since the needle engagement hole 74 is connected to the tube insertion hole 50, the air flowing through the supply tube flows into the air supply needle 66 inserted into the needle engagement hole 74.

Thereafter, the user attaches the vial attachment portion 12 of the vial adapter 10 to the vial 150. At this time, the vial 150 is in an inverted position. That is, the inlet/outlet of the vial 150 faces downward and the bottom portion 152 of the vial 150 faces upward. With this attachment, each one end portion of the air supply needle 66 and the liquid delivery tube 40 penetrates the plug member 154. In this state, when the user pushes the lever 304 by hand, a predetermined amount of air is compressed and discharged from the air supply port. When the user releases the hand from the lever 304, the lever 304 returns to the original position.

As described above, one end portion of the air supply needle 66 is positioned inside the vial 150. Therefore, the air compressed and discharged from the air supply port enters the vial 150 through the hollow interior of the air supply needle 66. As a result, the liquid medication L in the vial 150 is pushed by the air. As a result, a part of the liquid medication L passes through the hollow interior of the liquid delivery tube 40 and flows into the reservoir 102 from the liquid medication inlet 134. The user can visually check the amount of the liquid medication L stored in the reservoir 102 from the outside of the cartridge 120.

Even in a case where the liquid medication L in the reservoir 102 is moved along the cannula 132, the opening 136 and the second through-hole 128 are closed by the seal portion 56. Therefore, leakage of the liquid medication L through the opening 136 and the second through-hole 128 is avoided.

The user repeats pushing of the lever 304 as necessary. As a result, a predetermined amount of the liquid medication L is transferred from the vial 150 to the reservoir 102. In a case where the user checks that the amount of the liquid medication L has reached a target amount, the user ends the pushing of the lever 304. As a result, the movement of the liquid medication L from the vial 150 to the reservoir 102 is also ended.

Next, the user removes the liquid medication administration device 100 with the vial 150 attached to the vial attachment portion 12 of the vial adapter 10, from the loading slot 302. Thereafter, the user removes the vial 150 from the vial attachment portion 12. At this time, it is preferable to adopt an upright position in which the inlet/outlet of the vial 150 faces upward and the bottom portion 152 of the vial 150 faces downward.

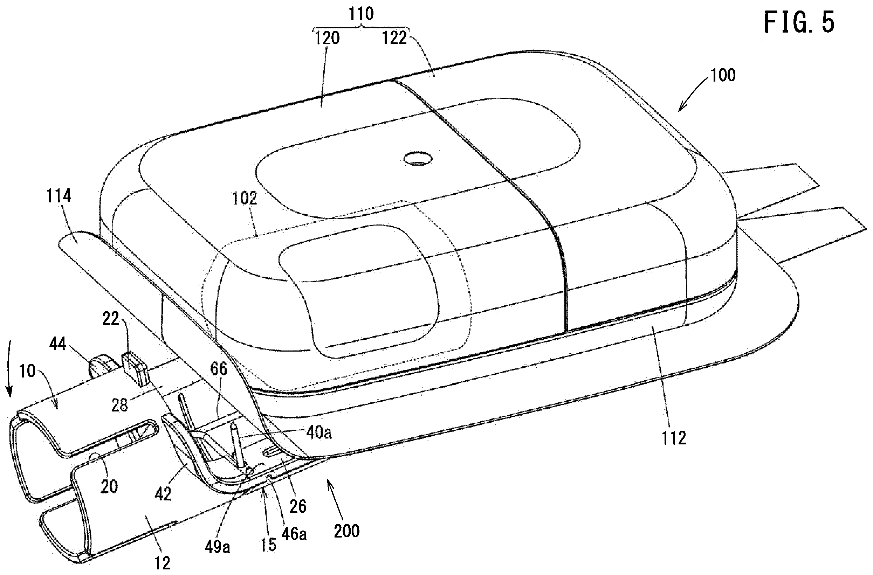

Next, as illustrated in Fig. 5, the user holds the vial attachment portion 12 of the vial adapter 10 with fingers in a state where the vial adapter 10 faces downward and the device main body 110 faces upward. The user further pulls down the vial attachment portion 12 in the downward direction. As described above, the bonding strength between the first engagement portion 32 and the second engagement portion 130 is not so large. Therefore, as the vial attachment portion 12 is pulled down, first, the first engagement protrusion 32a is detached from the first engagement hole 130a, and the second engagement protrusion 32b is easily detached from the second engagement hole 130b. In addition, the needle distal end 40a of the liquid delivery tube 40 is detached from the liquid medication inlet 134 and the first through-hole 124.

When the vial attachment portion 12 is further pulled down in the downward direction, the third engagement protrusion 32c and the fourth engagement protrusion 32d are detached from the third engagement hole 130c and the fourth engagement hole 130d, respectively. For the same reason as described above, this detachment also easily proceeds. In addition, the seal portion 56 is detached from the second through-hole 128. As described above, the attachment of the liquid medication administration device 100 to the device attachment portion 14 (the semi-cylindrical portion 15 and the socket portion 16) of the vial adapter 10 is released.

Next, the user peels off the release liner 114 from the adhesive sheet 112. As a result, the adhesive surface of the adhesive sheet 112 appears. The user affixes the adhesive surface to the skin of a predetermined part of the patient. Note that the abdomen is exemplified as the predetermined part. Thereafter, the user performs a predetermined operation for puncturing. The predetermined operation for puncturing is, for example, an operation of moving the cannula 132 of the device main body 110 using a puncture device different from the device main body 110. Alternatively, the predetermined operation for puncturing is an operation of pressing a puncture operation portion provided in the device main body 110. By the predetermined operation for puncturing, the cannula 132 advances from the opening 136 and the second through-hole 128 to puncture the patient's body.

Thereafter, a motor (not illustrated) accommodated in the pump unit 122 is driven. Accordingly, a plunger (not illustrated) provided inside the reservoir 102 starts to move. When the plunger is moved, the liquid medication L in the reservoir 102 is pushed by a gasket. Therefore, the liquid medication L flows out through the liquid medication outlet. The liquid medication L is moved along the tube, flows out of the cannula 132, and is administered to the patient.

In a case where the administration of the liquid medication L is ended, the device main body 110 is separated into the cartridge 120 to which the adhesive sheet 112 is affixed and the pump unit 122. The adhesive sheet 112 and the cartridge 120 are disposed of as medical waste. For example, after being charged, the pump unit 122 is attached to a new cartridge 120 in the same manner as in Fig. 4. That is, the pump unit 122 is reused.

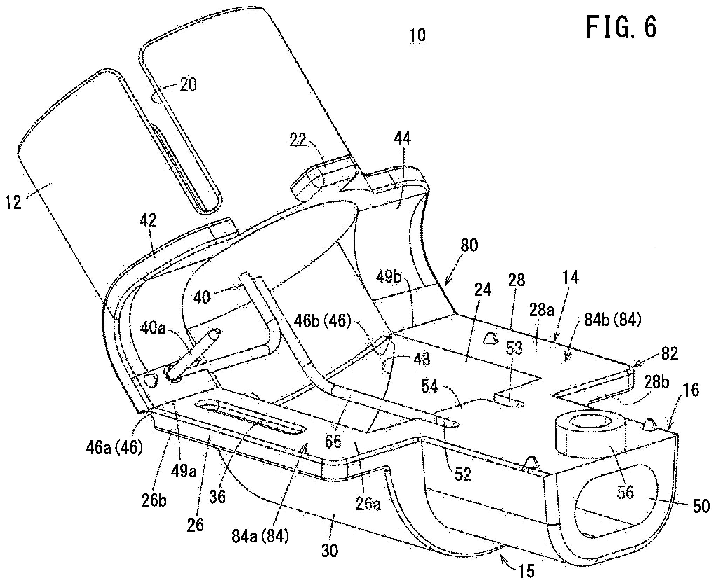

The vial adapter 10 from which the vial 150 and the liquid medication administration device 100 have been removed is discarded without being reused for hygiene reasons. At this time, the vial adapter 10 is folded with the first groove 46a and the second groove 46b (imaginary straight line M) as the folding points. Specifically, as illustrated in Fig. 6, the user holds the vial attachment portion 12 with fingers in a state where the outer surface of the peripheral wall portion 30 of the semi-cylindrical portion 15 is supported by the flat surface. Thereafter, the user pulls up the semi-cylindrical portion 15 in the upward direction. In the semi-cylindrical portion 15, a portion where the first groove 46a and the second groove 46b are formed is thinner than other portions. Moreover, the slit 48 is positioned between the first groove 46a and the second groove 46b. Therefore, as illustrated in Fig. 6, the semi-cylindrical portion 15 is easily folded with the first groove 46a, the slit 48, and the second groove 46b as starting points.

As a result, the first segment 80 approaches the second segment 82. Note that as the device attachment portion 14 is folded, the air supply needle 66 is also folded. In addition, the openings of the first groove 46a, the slit 48, and the second groove 46b become large.

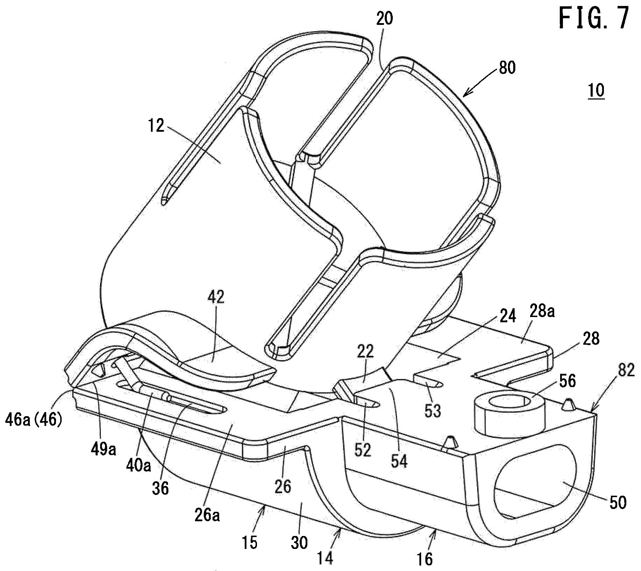

As the semi-cylindrical portion 15 is further folded, as illustrated in Fig. 7, the needle distal end 40a (protrusion portion) of the liquid delivery tube 40 enters the recess portion 36 formed in the first flat portion 26 of the device attachment portion 14. This prevents the needle distal end 40a from protruding outward from between the first curved portion 42 and the first flat portion 26. The first curved portion 42 covers the liquid delivery tube 40. That is, the needle distal end 40a, which is the protrusion portion of the liquid delivery tube 40, is shielded by the first flat portion 26 and the first curved portion 42. This shielding prevents the protrusion portion of the liquid delivery tube 40 from coming into contact with the user. Therefore, the user can easily handle the vial adapter 10.

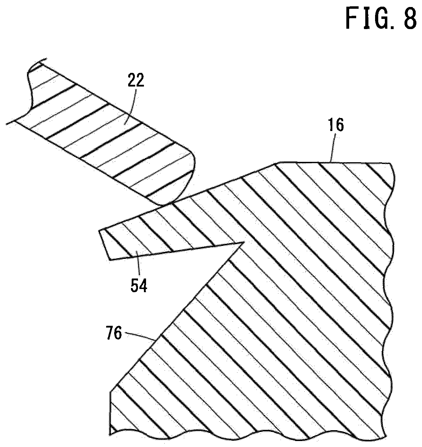

When the semi-cylindrical portion 15 is folded, as illustrated in Fig. 8, the first stopper portion 22 provided in the vial attachment portion 12 abuts on the second stopper portion 54. Due to this contact, the second stopper portion 54 is pushed down and bent downward. When the semi-cylindrical portion 15 is further folded, the first stopper portion 22 is lowered toward the recessed space 76 and passes beyond the second stopper portion 54. As a result, as illustrated in Fig. 7, the second stopper portion 54 returns to the original position by elasticity. As a result, the first stopper portion 22 is engaged with the second stopper portion 54 in a state of being inserted into the recessed space 76.

Therefore, it is difficult for the first stopper portion 22 to return to the original position beyond the second stopper portion 54. That is, it is difficult for the first segment 80 to be separated from the second segment 82. In other words, engagement of the first stopper portion 22 with the second stopper portion 54 prevents the vial adapter 10 from returning to the shape before being folded.

The folded vial adapter 10 is more compact than the vial adapter 10 before being folded. That is, since the first stopper portion 22 is engaged with the second stopper portion 54, the vial adapter 10 maintains the compact state. Therefore, even in a case where a plurality of vial adapters 10 is discarded at the same time, the medical waste in which the plurality of vial adapters 10 is accumulated is also compact. In other words, the formation of bulky medical waste is avoided. Therefore, the discarded vial adapter 10 can be stored in a small storage space. This may avoid the vial adapter 10 occupying a large space between discarding and disposing of the vial adapter 10.

Note that the present invention is not limited to the above disclosure, and various configurations can be adopted without departing from the gist of the present invention.

## Claims

1. A vial adapter (10) for transferring liquid medication (L) in a vial (150) to a reservoir (102) of a liquid medication administration device (100), the vial adapter comprising:
a vial attachment portion (12) to which a vial is attached;
a device attachment portion (14) that extends from the vial attachment portion and to which the liquid medication administration device is attached;
a liquid delivery tube (40) that has a protrusion portion (40a) protruding from the device attachment portion and delivers the liquid medication from the vial to the reservoir; and
a groove (46) that is formed in the device attachment portion and extends in a direction orthogonal to an attachment direction of the vial to the vial attachment portion,
wherein the device attachment portion is able to be folded with the groove as a folding point, and
when the device attachment portion is folded, the protrusion portion of the liquid delivery tube is shielded by the device attachment portion.

2. The vial adapter according to claim 1,
wherein when the vial adapter is divided, with the groove as a boundary, into a first segment (80) including the vial attachment portion and a second segment (82) not including the vial attachment portion, the first segment includes a first stopper portion (22) and the second segment includes a second stopper portion (54), and
when the device attachment portion is folded such that the first segment approaches the second segment, the first stopper portion and the second stopper portion are engaged with each other.

3. The vial adapter according to claim 1,
wherein the vial adapter includes an air supply needle (66) for supplying air to the vial, the device attachment portion includes a peripheral wall portion (30) covering the air supply needle, and a slit (48) continuous with the groove is formed in the peripheral wall portion.

4. The vial adapter according to claim 1, further comprising:
a recess portion (36) that accommodates the protrusion portion of the liquid delivery tube when the device attachment portion is folded.

5. The vial adapter according to claim 1,
wherein the device attachment portion includes an abutting portion (84) that abuts on the liquid medication administration device, and the groove is formed in the abutting portion.

6. The vial adapter according to claim 5,
wherein the abutting portion includes a flat portion (26, 28) and a curved portion (42, 44), and
when the device attachment portion is folded, the flat portion and the curved portion face each other, and the curved portion covers the protrusion portion of the liquid delivery tube.

7. The vial adapter according to claim 1,
wherein the device attachment portion includes a protrusion for engagement.

8. The vial adapter according to any one of claims 1 to 7,
wherein the device attachment portion includes a seal portion (56) formed to cover an opening (136) that is formed in the liquid medication administration device and is for projecting a cannula (132) to puncture a patient.

9. A cartridge unit (200) comprising:
a vial adapter (10); and
a cartridge (120) constituting a liquid medication administration device (100) in which a reservoir (102) is accommodated,
wherein the vial adapter includes
a vial attachment portion (12) to which a vial (150) containing liquid medication (L) is attached,
a device attachment portion (14) that extends from the vial attachment portion and to which the cartridge is attached,
a liquid delivery tube (40) that has a protrusion portion (40a) protruding from the device attachment portion and delivers the liquid medication from the vial to the reservoir,
a groove (46) that is formed in the device attachment portion and extends in a direction orthogonal to an attachment direction of the vial to the vial attachment portion, and
a first engagement portion (32) provided in the device attachment portion,
the cartridge includes a second engagement portion (130),
one of the first engagement portion and the second engagement portion is an engagement protrusion (32a to 32d),
the other of the first engagement portion and the second engagement portion is an engagement hole (130a to 130d), and
the engagement protrusion is inserted into the engagement hole.

10. The cartridge unit according to claim 9,
wherein the first engagement portion and the second engagement portion are bonded to each other.

11. The cartridge unit according to claim 10,
wherein the first engagement portion and the second engagement portion are welded to each other.

12. The cartridge unit according to claim 9,
wherein the cartridge includes a cannula (132) that punctures a patient to deliver the liquid medication from the reservoir to the patient, and an opening (136) for projecting the cannula, and
the device attachment portion includes a seal portion (56) that covers the opening when the engagement protrusion is inserted into the engagement hole.
